# EUROPEAN PATENT APPLICATION

(11) **EP 4 296 264 A1**
(43) Date of publication of application: **27.12.2023**
(21) Application number: 23768101.0
(22) Date of filing: 17.02.2023
(51) Int. Cl.: C07C 319/28, C07C 321/26

(54) **1,6-NAPHTHALENEDITHIOL AND METHOD FOR PRODUCING SAME**

(30) Priority: 14.03.2022 JP 2022039384
(71) Applicant: SUGAI CHEMICAL INDUSTRY CO., LTD., Wakayama-shi, Wakayama 6410043 (JP)
(72) Inventor: MINEYAMA Kenji, Wakayama-shi, Wakayama 6410043 (JP); KAZEKAMI Yutaka, Wakayama-shi, Wakayama 6410043 (JP); FUJIHARA Hiroki, Wakayama-shi, Wakayama 6410043 (JP)
(74) Representative: Grünecker Patent- und Rechtsanwälte PartG mbB
(86) International application number: PCT/JP2023/005626
(87) International publication number: WO 2023/176299

(57) **Abstract**

Provided are a naphthalenedithiol (or a naphthalenedithiol product) having a high purity and a reduced coloration, and a process for producing the same.

A 1,6-naphthalenedithiol (a 1,6-naphthalenedithiol product) according to the present invention has a purity of not less than 99.5% and a polymeric 1,6-naphthalenedithiol content of not more than 0.2%, each determined by high performance liquid chromatography using area normalization; and has a Gardner color scale in a molten state of not more than 1. The polymeric 1,6-naphthalenedithiol may be a disulfide form as a dimeric 1,6-naphthalenedithiol. Such a 1,6-naphthalenedithiol product can be produced by distilling a crude raw material containing 1,6-naphthalenedithiol and at least a polymeric 1,6-naphthalenedithiol to obtain a distillate fraction containing 1,6-naphthalenedithiol. The crude raw material may be a dry solid of a crudely purified product of 1,6-naphthalenedithiol from a reaction mixture.

## Description

### TECHNICAL FIELD

The present invention relates to a 1,6-naphthalenedithiol (or a 1,6-naphthalenedithiol product) having a high purity and less or no coloration, and a process for producing the same.

### BACKGROUND ART

A naphthalenedithiol or a derivative thereof is used as a functional material, or a raw material or a reaction intermediate thereof in various fields including an optical material, an electric and electronic material, and a medicine.

Japanese Patent Application Laid-Open Publication No. 2008-527413 (JP 2008-527413 A, Patent Document 1) discloses a display including a light transmissible substrate and a specified hardcoat layer bonded to the substrate. Example 12 of this document discloses that 2,7-dihydroxynaphthalene is allowed to react with dimethylthiocarbamoyl chloride, the produced dimethyl-thiocarbamic acid O-(7-dimethylthiocarbamoyloxy-naphthalen-2-yl) ester is dried and heated and is then cooled, the solid matter is recrystallized from ethyl acetate to obtain dimethyl-thiocarbamic acid S-(7-dimethylcarbamoylsulfanyl-naphthalen-2-yl) ester, this ester is acidified with hydrochloric acid, and the solid matter is filtered and dried to obtain 2,7-naphthalenedithiol.

U.S. Patent No. 2463219 specification (Patent Document 2) discloses a method for improving a processability of a synthetic elastomer, the method comprising adding an arylmercaptan and oxygen at a specific ratio to a latex of a synthetic elastomer containing a 1,3-butadiene-styrene copolymer prepared by emulsion polymerization. This document describes 2,5-dimercapto-naphthalene (1,6-naphthalenedithiol) as an example of the arylmercaptan.

U.S. Patent No. 9170495 specification (Patent Document 3) refers to a polymer for forming a resist under-layer, and Synthesis Examples 10 and 11 of this document disclose that such a polymer is obtained by allowing 9-fluorenone or thioxanthone to react with 1,6-naphthalenedithiol in the presence of sulfuric acid, and allowing the produced phenol monomer to react with formaldehyde.

Toshio Nambara, "Studies on Chemotherapeutics for Acid-fast Bacilli. XIII. On Some Compounds Related to Vinyl Sulfone and their Bacteriostatic and Chemical Activities. (2)", YAKUGAKU ZASSHI, 1955, Vol. 75, issue 12, p. 1560-1564 (Nonpatent Document 1) discloses in Fig. 1 that 1,5-bis(2-(C₁₋₂alkoxy)ethylsulfonyl)naphthalenes are synthesized from naphthalene-1,5-dithiol through 1,5-bis(2-hydroxyethylthio)naphthalene, 1,5-bis(2-chloroethylthio)naphthalene, and 1,5-bis(2-chloroethylsulfonyl) naphthalene.

### CITATION LIST

### PATENT LITERATURE

Patent Document 1: JP 2008-527413 A (Claims 1 and 6, [0031], [0038], and Example 12)
Patent Document 2: U.S. Patent No. 2463219 specification (Claim 1, and column 5, line 12)
Patent Document 3: U.S. Patent No. 9170495 specification (Reaction Scheme 1, and Synthesis Examples 10 and 11)

### NONPATENT LITERATURE

Nonpatent Document 1: Toshio Nambara, "Studies on Chemotherapeutics for Acid-fast Bacilli. XIII. On Some Compounds Related to Vinyl Sulfone and their Bacteriostatic and Chemical Activities. (2)", YAKUGAKU ZASSHI, 1955, Vol. 75, issue 12, p. 1560-1564 (Fig. 1, Experimental, and others)

### SUMMARY OF INVENTION

### TECHNICAL PROBLEM

However, these documents fail to describe characteristics of 1,6-naphthalenedithiol. Moreover, the naphthalenedithiol is liable to coloration, and easily produces a polymeric substance, including a disulfide, due to highly active mercapto groups. In addition, the introduction of an arene ring, particularly a fused or condensed polycyclic arene ring such as a naphthalene ring, to a chemical structure thereof typically increases heat resistance (melting point or boiling point) and refractive index, while showing a tendency to decrease dissolubility. Thus, the naphthalenedithiol is significantly limited in uses.

It is therefore an object of the present invention to provide a naphthalenedithiol (or a naphthalenedithiol product) having a high purity and a reduced coloration, and a process for producing the same.

Another object of the present invention is to provide a naphthalenedithiol (or a naphthalenedithiol product) having a high refractive index compatible with a high dissolubility, and a process for producing the same.

### SOLUTION TO PROBLEM

The inventors of the present invention focused on a naphthalenedithiol with various positional isomers and made intensive studies to achieve the above objects, and finally found that, among various naphthalenedithiol positional isomers, 1,6-naphthalenedithiol (1,6-form) has unique properties different from the other positional isomers, specifically a high refractive index in spite of a low melting point, and a high dissolubility. The inventors made further intensive studies based on these findings, and finally found that a crude raw material containing 1,6-naphthalenedithiol (1,6-form) and a polymeric form thereof is distilled or evaporated to obtain a 1,6-naphthalenedithiol (1,6-form) product having an extremely high purity and less or no coloration. The present invention was accomplished based on the above findings. The present invention may include the following aspects [1] to [8].

That is, the present invention includes [1] a 1,6-naphthalenedithiol (or a 1,6-naphthalenedithiol product) having a purity of not less than 99.5% and a polymeric 1,6-naphthalenedithiol content of not more than 0.2%, each determined by high performance liquid chromatography using area normalization. The 1,6-naphthalenedithiol (or the 1,6-naphthalenedithiol product) is hardly colored (or substantially colorless) and has a Gardner color scale in a molten state of not more than 1. In the aspect [1], [2] the polymeric 1,6-naphthalenedithiol may be a disulfide (a disulfide form) as a dimeric 1,6-naphthalenedithiol.

The present invention also includes [3] a process for producing a 1,6-naphthalenedithiol product (a 1,6-naphthalenedithiol product with a high purity and having a reduced coloration) from a crude raw material containing 1,6-naphthalenedithiol and at least a polymeric 1,6-naphthalenedithiol. In the process, the crude raw material is distilled to obtain the 1,6-naphthalenedithiol product.

In the process of the aspect [3], [4] the crude raw material may be a crudely purified product of 1,6-naphthalenedithiol from a reaction mixture which is obtained in synthesis of 1,6-naphthalenedithiol. In the process of the aspect [3] or [4], [5] the crude raw material may be a dry solid of a crudely purified product of 1,6-naphthalenedithiol from a reaction mixture which is obtained in synthesis of 1,6-naphthalenedithiol. In the process of any one of the aspects [3] to [5], [6] the crude raw material may be subjected to a distillation under a reduced pressure or a vacuum distillation to obtain (or withdraw) a distillate fraction containing 1,6-naphthalenedithiol. In the process of any one of the aspects [3] to [6], [7] the crude raw material may be distilled at a pressure of 0.1 to 30 hPa to obtain (or withdraw) a distillate fraction having a temperature of 140 to 212°C. For example, in the process of any one of the aspects [3] to [7], [8] the 1,6-naphthalenedithiol product may be produced by subjecting the dry solid of the crudely purified product as the crude raw material to a simple distillation to obtain (or withdraw) a distillate fraction containing 1,6-naphthalenedithiol from an upper position of a distillation apparatus.

### ADVANTAGEOUS EFFECTS OF INVENTION

The 1,6-naphthalenedithiol (or the 1,6-naphthalenedithiol product) according to the present invention has an extremely low content of a polymeric form such as a disulfide form of 1,6-naphthalenedithiol and has a high purity with less or no coloration, even though 1,6-naphthalenedithiol has mercapto groups. In addition, the 1,6-naphthalenedithiol product shows a high dissolubility and has a high refractive index compatible with a high dissolubility, even though 1,6-naphthalenedithiol has a naphthalene ring. Further, the 1,6-naphthalenedithiol product having a high purity and a significantly reduced coloration can be produced by distillation, which is a simple operation. Such a 1,6-naphthalenedithiol product can be used as, for example, a raw material or reaction intermediate for a functional material (such as an optical material) and/or others.

### DESCRIPTION OF EMBODIMENTS

[1,6-Naphthalenedithiol]

1,6-Naphthalenedithiol (which may simply be referred to as 1,6-form hereinafter) represented by the following formula (1) is a positional isomer of naphthalenedithiol and shows unique properties as below.

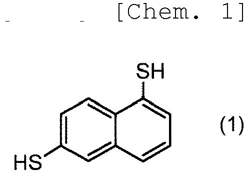

Specifically, 2,7-naphthalenedithiol (2,7-form) described in the Patent Document 1 has a melting point of 186 to 188°C, 2,6-naphthalenedithiol (2,6-form) has a melting point of 196 to 197°C, and 1,5-naphthalenedithiol (1,5-form) described in the Nonpatent Document 1 has a melting point of 120 to 121°C. Thus, these positional isomers of naphthalenedithiol each have a high melting point and are expected to show boiling point elevation due to hydrogen bonds of mercapto groups. Further, heat stability data by differential scanning calorimetry (DSC) show that 1,5-form, 2,6-form, and 2,7-form decrease by 5% or more in weight at 176°C, 196°C, and 188°C, respectively. Thus, it is technically difficult to adopt purification of these naphthalenedithiols by distillation at a relatively high temperature. For example, when 1,5-form, 2,6-form, and 2,7-form are tried to be distilled under vacuum, an outflow (or exit) line is to be maintained at a high temperature in order to prevent crystallization. However, such a high temperature of the outflow line causes not only easy re-evaporation of the naphthalenedithiols in the outflow line but also crystallization and clogging of the naphthalenedithiols in a subsequent line, thus making it impossible to operate the distillation.

In addition, since 1,5-form, 2,6-form, and 2,7-form have a low dissolubility in an organic solvent, these forms maintain the crystal state even after mixing with an organic solvent. Thus, the purity of the naphthalenedithiols cannot be increased by crystallization.

It may also be possible (or contemplated) to purify the naphthalenedithiols by column chromatography. However, the purification by column chromatography is unsuitable for the industrial production of the naphthalenedithiols. In addition, even when the purification by column chromatography is carried out, the resulting naphthalenedithiol products are still significantly colored. It is thus difficult to obtain naphthalenedithiol products having colorless, or less or no coloration.

Under these circumstances, further studies on the positional isomers have found that 1,6-naphthalenedithiol (1,6-form) has unique properties. Specifically, the studies have found that 1,6-form is characterized by an extremely low melting point of 35 to 36°C compared with the other positional isomers, and a high refractive index and a high dissolubility in an organic solvent compared with the other positional isomers. Based on these properties, the inventors have further investigated on the purification method of 1,6-form but have failed to obtain a 1,6-form product having an improved purity and a reduced coloration even when a crude raw material containing 1,6-naphthalenedithiol (1,6-form) is purified by crystallization. The investigation of the cause (or factors) of this failure has revealed that usually naphthalenedithiol is contaminated or coexists with a polymeric naphthalenedithiol including a dimeric form (a disulfide form), such a polymeric form typically has a low dissolubility compared with naphthalenedithiol, and especially a trimeric or higher polymeric form is hardly dissolved in an organic solvent. Thus, it has been revealed that, even if 1,6-form is tried to be purified by crystallization, the polymeric naphthalenedithiol is preferentially crystallized and precipitated to 1,6-form and fails to be separated from 1,6-form, and not only the dimeric form (the disulfide), which may have a similar dissolubility to 1,6-form, but also a colored component cannot effectively be removed. Further, it has also been revealed that 1,6-form, which has a high solubility in a solvent, is hardly precipitated as a crystal, and even 1,6-form precipitated has a greatly lowered yield due to an extremely large loss.

Further, in spite of studies on the purification of 1,6-form by adsorption treatment with an activated carbon and others, none of adsorption treatments can remove the colored component, and thus it is difficult to obtain a 1,6-form product having a high purity with less or no coloration.

Moreover, focusing on the fact that the dimeric form (disulfide form) and trimeric or higher polymeric form as by-products hardly vaporize, the inventors have purified 1,6-form by distillation and have unexpectedly found that the distillation can be carried out at a relatively low temperature to obtain a 1,6-form product with a high purity. In particular, it has been unexpectedly found that the colored component which failed to be removed even by column chromatography can effectively be removed and that thus purified 1,6-form product has less or no coloration. The present invention has been accomplished by further studies based on these findings.

That is, the 1,6-naphthalenedithiol (or the 1,6-naphthalenedithiol product) according to the present invention has a purity of not less than 99.5% (99.5 to 100%), preferably not less than 99.7% (99.7 to 100%), and further preferably not less than 99.8%, determined by high performance liquid chromatography (HPLC) using area normalization.

Further, a polymeric 1,6-naphthalenedithiol content of the 1,6-naphthalenedithiol product is not more than 0.2%, preferably not more than 0.15%, further preferably not more than 0.1%, particularly not more than 0.05%, and further preferably less than a detection limit, determined by high performance liquid chromatography (HPLC) using area normalization. The above-mentioned polymeric form (polymeric 1,6-naphthalenedithiol) may be a dimeric 1,6-naphthalenedithiol (a disulfide or a disulfide form). In other words, the dimeric form (the disulfide form) content may be the above-mentioned proportion.

As described above, the dimeric form (the disulfide form) seems to show a high dissolubility, as well as 1,6-form. In contrast, the trimeric or higher polymeric form seems to be hardly dissolved in an organic solvent. Thus, it is usually difficult to quantitatively determine the trimeric or higher polymeric form by HPLC.

Moreover, the 1,6-naphthalenedithiol (or the 1,6-naphthalenedithiol product) according to the present invention has a Gardner color scale in a molten state of not more than 1. Since 1,6-naphthalenedithiol has a melting point of about 35 to 36°C, heating to a temperature (a temperature of not lower than 40°C, particularly about 45 to 55°C) of not lower than the melting point allows rapid measurement of the Gardner color scale in a molten state. The Gardner color scale is determined by visually comparing Gardner color scale standard solutions defined in Japanese Industrial Standards (JIS) K 0071:1998 "Testing methods for color of chemical products- Part 2: Gardner color scale" with a transparent color of a sample, and evaluating the degree of the coloration of the sample as a color scale number of a Gardner color scale standard solution having a color corresponding to the transparent color (color intensity) of the sample. In such a Gardner color scale, the 1,6-form (or the 1,6-form product) according to the present invention has the lowest Gardner color scale of 1, or a lower color scale (a Gardner color scale of less than 1).

The purified 1,6-naphthalenedithiol (or 1,6-naphthalenedithiol product) mentioned above may be in a crystalline form at a room temperature (20°C). The 1,6-naphthalenedithiol having a crystalline form may have a melting point of about 35 to 36°C.

### [Process for producing 1,6-naphthalenedithiol]

### [Synthesis or preparation of 1,6-naphthalenedithiol]

In the present invention, a process for synthesizing or preparing 1,6-naphthalenedithiol or 1,6-form (or a 1,6-naphthalenedithiol product) is not particularly limited to a specific one, and the present invention can be applied to a reaction product containing 1,6-form obtained by various processes. Examples of the process for synthesizing or preparing 1,6-form may include a known process for synthesizing naphthalenedithiol such as a process which comprises using 1,6-naphthalenedisulfonic acid or a salt thereof as a raw material.

In this process, 1,6-naphthalenedithiol (1,6-form) may be prepared by (1) allowing 1,6-naphthalenedisulfonic acid or a salt thereof to react with a halogenating agent to prepare a 1,6-bis(halosulfonyl)naphthalene and (2) reducing the produced 1,6-bis(halosulfonyl)naphthalene in the presence of a reducing agent.

In the above-mentioned halosulfonylation reaction (1), examples of a salt of 1,6-naphthalenedisulfonic acid may include an alkali metal salt and an ammonium salt. A preferred salt of 1,6-naphthalenedisulfonic acid includes an alkali metal salt (a potassium salt, preferably a sodium salt).

As examples of the halogenating agent, there may be mentioned phosgene, phosphorus pentachloride, phosphorus trichloride, phosphorus oxychloride, sulfuryl chloride, oxalyl chloride, and thionyl chloride. The halogenating agent may be used alone or in combination of two or more. A preferred halogenating agent includes a chlorinating agent such as thionyl chloride. The ratio of the halogenating agent relative to 1 mol of the salt of 1,6-naphthalenedisulfonic acid may, for example, be about 2 to 10 mol, preferably about 2.1 to 5 mol, and further preferably about 2.3 to 3 mol.

The reaction may be carried out in the presence or absence of a catalyst. Examples of the catalyst may include an amide such as N,N-dimethylformamide (DMF) and N,N-dimethylacetamide (DMAc) (preferably DMF); a pyridine such as pyridine and 4-(N,N-dimethylamino)pyridine; and a tertiary amine such as triethylamine. The catalyst may be used alone or in combination of two or more. A preferred catalyst includes an amide such as DMF. The ratio of the catalyst relative to 1 mol of the salt of 1,6-naphthalenedisulfonic acid may, for example, be about 0.01 to 10 mol, preferably about 0.01 to 1 mol, further preferably about 0.1 to 0.7 mol, and particularly about 0.2 to 0.5 mol. The amide such as DMF and the chlorinating agent such as thionyl chloride may form a Vilsmeier complex for chlorosulfonylation.

The reaction may be carried out in the presence or absence of a solvent. Examples of the solvent may include an inert or inactive solvent to the reaction, for example, a hydrocarbon such as hexane, cyclohexane, toluene, and xylene, a halogenated hydrocarbon such as dichloromethane, chloroform, and 1,2-dichloroethane, an ester such as methyl acetate and ethyl acetate, a ketone such as methyl ethyl ketone and methyl isobutyl ketone, an ether such as tetrahydrofuran and dioxane, a nitrile such as acetonitrile and propionitrile, an amide such as DMF and DMAc, and a sulfoxide such as dimethyl sulfoxide. The solvent may be used alone or as a mixed solvent in combination of two or more. Examples of a preferred solvent include an aromatic hydrocarbon such as toluene and xylene. The ratio of the solvent is not particularly limited to a specific ratio, and may, for example, be about 10 to 1000 parts by mass, preferably about 100 to 800 parts by mass, and further preferably about 300 to 600 parts by mass, relative to 100 parts by mass of the salt of 1,6-naphthalenedisulfonic acid.

The reaction temperature may, for example, be about 50 to 120°C, preferably about 80 to 110°C, and further preferably about 90 to 100°C. The reaction time may, for example, be about 1 to 24 hours, preferably about 2 to 18 hours, and further preferably about 4 to 12 hours.

The reaction may be carried out under an atmosphere of an air or an inert gas (such as nitrogen gas; and a rare or noble gas such as argon and helium) and preferably under an inert gas atmosphere. The reaction may be carried out under a normal or ordinary pressure, under an applied pressure, or under a reduced pressure.

After the completion of the reaction, the reaction product may be separated with purification by a conventional separation-purification means such as washing, extraction, concentration, filtration, crystallization, centrifugal separation, column chromatography, activated carbon treatment, and drying, and a combination of these means; or may directly be subjected (such as, in a solution state) to the above-mentioned reduction reaction (2) without separation-purification. A preferred process may include performing the following operations at least once or repeating multiple times: adding water to a reaction mixture while stirring to deactivate the halogenating agent, and separating the resulting mixture into an organic phase and an aqueous (or water) phase. The resulting organic phase may optionally be separated with purification by an operation such as drying, solidification by drying, distillation, and crystallization and then may be subjected to the succeeding reduction reaction. In a preferred embodiment, the resulting organic phase may be subjected to the succeeding reduction reaction (2) .

In the above-mentioned reduction reaction (2), the species (or kind) of the reducing agent is not particularly limited to a specific one and may include, for example, a combination of zinc and an acid, a combination of tin and an acid, a combination of tin chloride and an acid, lithium aluminum hydride, lithium borohydride, and sodium borohydride. A preferred reducing agent includes a combination of zinc (such as zinc powder) and an acid.

The acid may include, for example, an inorganic acid such as hydrochloric acid and sulfuric acid, and is preferably hydrochloric acid. The acid may be used alone or in combination of two or more. The ratio of the acid relative to 1 mol of the 1,6-bis(halosulfonyl)naphthalene may, for example, be about 10 to 100 mol, preferably about 40 to 60 mol, and further preferably about 45 to 55 mol. The acid may be used in the form of an aqueous solution.

The ratio of the reducing agent relative to 1 mol of the 1,6-bis(halosulfonyl)naphthalene may, for example, be about 5 to 20 mol, preferably about 8 to 17 mol, and further preferably about 10 to 15 mol.

The reaction may be carried out in the presence of a solvent. The solvent may include the same as the solvent in the above-mentioned halosulfonylation reaction (1). The solvent may be used alone or as a mixed solvent in combination of two or more. In a case where the acid is used, water contained in the form of the aqueous solution may also be used as the solvent. In particular, the reduction reaction (2) is preferably carried out in the presence of water liquid-separable from the reaction solvent (such as a hydrophobic aromatic hydrocarbon) after the completion of the reaction. The amount of the solvent is not particularly limited to a specific one, and may, for example, be about 100 to 5000 parts by mass and preferably about 500 to 4000 parts by mass, relative to 100 parts by mass of the 1,6-bis(halosulfonyl)naphthalene.

The reaction temperature may, for example, be about 30 to 120°C, preferably about 40 to 100°C, and further preferably about 50 to 80°C. The reaction time may, for example, be about 0.5 to 24 hours, preferably about 1 to 12 hours, and further preferably about 2 to 5 hours.

The reaction may be carried out in the air and may preferably be carried out under an atmosphere of an inert gas (such as nitrogen gas; and a rare or noble gas such as argon and helium). The reaction may be carried out under a normal or ordinary pressure, under an applied pressure, or under a reduced pressure.

In a case where the reduction reaction (2) is carried out in the presence of water liquid-separable from the reaction solvent, 1,6-form can advantageously be transferred to an organic phase, and thus the separation-purification efficiency and separation accuracy of 1,6-form can be improved.

### [Preparation and distillation of crude raw material]

The present invention includes a process including distilling a crude raw material containing 1,6-form and at least a polymeric 1,6-form to produce 1,6-form (or a 1,6-form product). The polymeric form contained in the crude raw material may be a dimeric form (a disulfide form), a trimeric form, or a higher polymeric form.

Such a crude raw material may be a reaction mixture which is obtained in synthesis (or preparation) of 1,6-form. In the distillation of such a reaction mixture, a light component such as an organic solvent is distilled from an upper position or overhead (or column top) of a distillation apparatus, while a heavy component containing 1,6-form and a polymeric form thereof remains in a lower position or bottom (column bottom) of the distillation apparatus. In practical cases, the heavy component further contains an impurity such as a metal component (such as a catalyst component and an inorganic base). Thus, in a case where the reaction mixture is tried to be directly distillated for separation-purification of 1,6-form, the separation efficiency of 1,6-form is decreased and the load on the distillation apparatus is increased.

Thus, the crude raw material is preferably a crudely or roughly purified product obtained by crudely purifying the reaction product (1,6-form) from the reaction mixture. After the completion of the reaction, it is preferred to separate the reaction mixture into the crude raw material (the crudely purified product) mainly containing 1,6-form and a mixture of the other components in the crude purification, and to distill the crude raw material. The reaction mixture may be concentrated if necessary.

In such a crude purification, depending on the characteristics (such as evaporation characteristics, liquid separability, liquid characteristics such as alkalinity, and precipitability) of the reaction mixture or concentrated reaction mixture, there may be used various separation-purification means (or treatments) capable of separating-purifying the reaction product (1,6-form), such as distillation, washing, liquid-liquid separation (liquid-liquid extraction or liquid separation) or extraction, concentration, neutralization, crystallization or precipitation, solid-liquid separation (such as filtration and centrifugal separation), column chromatography, activated carbon treatment, drying, or solidification by drying. These separation-purification means may be combined to crudely purify the reaction product (1,6-form) (or to separate the reaction product with purification). For example, as described above, the reaction mixture may be distilled to obtain the heavy component from the lower position or column bottom of the distillation apparatus, and the heavy component as the raw material may further be distilled by the or another distillation apparatus. However, as described above, the purification with these separation-purification means has a lowered separation efficiency of 1,6-form, and it is difficult to produce a 1,6-form product having a high purity and a reduced coloration by a simple operation.

In a preferred process, in order to reduce impurities contaminated in the crude raw material (the crudely purified product) containing the reaction product (1,6-form), the reaction mixture is subjected to crude purification, and the crudely purified product is distilled. The crudely purified product can be obtained by, for example, a separating operation such as liquid-liquid separation (liquid-liquid extraction) or extraction, concentration, crystallization or precipitation, solid-liquid separation (such as filtration and centrifugal separation), drying, or solidification by drying, and a combination of these separating operations. As a method for obtaining a preferred crudely purified product, there may be used at least one separation-purification means selected from liquid-liquid separation (or extraction or liquid-liquid phase separation) and precipitation (or crystallization).

For the liquid-liquid separation (or extraction), there may be used a solvent (a poor solvent) having a low dissolubility to the reaction product (1,6-form), together with an organic solvent (a good solvent) having a high dissolubility to the reaction product (1,6-form). The reaction mixture contains at least the good solvent, and the poor solvent may be added to the reaction mixture to separate the reaction mixture into an organic phase containing 1,6-form and an aqueous phase, alternatively the reaction may be carried out in the reaction system containing both good solvent and poor solvent to separate (or liquid-separate) the reaction mixture into an organic phase and an aqueous phase. In the latter embodiment, 1,6-form can be extracted or distributed to the organic phase with the liquid-liquid separation, and thus, it is advantageous from the point of view of the reaction operation.

Examples of the good solvent may include an alcohol such as methanol and ethanol, an aliphatic hydrocarbon such as hexane and octane, an alicyclic hydrocarbon such as cyclohexane and methylcyclohexane, an aromatic hydrocarbon such as toluene and xylene, a halogenated hydrocarbon such as dichloromethane, chloroform, and 1,2-dichloroethane, an ester such as methyl acetate and ethyl acetate, and a ketone such as methyl ethyl ketone and methyl isobutyl ketone. Such a good solvent may be used alone or as a mixed solvent in combination of two or more. A preferred good solvent includes an aromatic hydrocarbon such as toluene and xylene.

As examples of the poor solvent, there may be mentioned water. A preferred poor solvent includes water.

The separated organic phase (the organic phase containing the extracted 1,6-form) may be used as a crudely (or roughly) purified product as it is; the separated organic phase may be filtered to obtain a filtrate which is used as a crudely purified product, or if necessary, a combination of the filtrate and a washed solution may be used as a crudely purified product, wherein the washed solution is obtained by washing the residue (or cake) of the filtration; or the separated organic phase may be concentrated to give a concentrate which is used as a crudely purified product.

In the crystallization (or precipitation) operation, the reaction mixture may be concentrated for crystallization (or precipitation); the reaction mixture (a reaction mixture containing at least the poor solvent and forming a homogeneous reaction system at a reaction temperature) may be subjected to cooling crystallization, or the poor solvent may be added to the reaction mixture for crystallization. In order to form a homogeneous system in the reaction, the reaction mixture contains at least the good solvent practically, and thus, it is preferred to add the poor solvent to the reaction mixture for precipitating or crystallizing a precipitation (or sedimentation) product containing 1,6-form from the reaction mixture.

Examples of the good solvent may include, in addition to the same good solvent as described above, a ketone including acetone, an ether such as tetrahydrofuran and dioxane, a nitrile such as acetonitrile and propionitrile, an amide such as DMF and DMAc, and a sulfoxide such as dimethyl sulfoxide. Such a good solvent may also be used alone or in combination of two or more. As examples of a preferred good solvent, there may be mentioned an aromatic hydrocarbon such as toluene and xylene. Examples of the poor solvent (crystallization solvent) may include the same poor solvent as described above. As examples of a preferred poor solvent, there may be mentioned water, methanol, and ethanol. The poor solvent preferably contains at least water.

In a further preferred process, utilizing a significant difference in dissolubility and precipitability of 1,6-form in these solvents (in the poor solvent such as water, and in the good solvent such as a hydrocarbon), the crudely purified product can be obtained by a liquid-liquid separation operation of separating the reaction mixture into an organic phase (or an extraction phase) and an aqueous phase, and/or a filtration operation of separating a precipitation product crystallized (or precipitated) from the reaction mixture (the reaction system) by a solid-liquid separation (containing washing of a residue). Specifically, the crudely purified product may be a separated organic phase, may be a filtrate obtained by filtration of the organic phase, or may optionally be a combination of the filtrate and a washed solution which is obtained by washing the residue (or cake) of the filtration, or if necessary, the organic phase may be a concentrate concentrated to a predetermined concentration; the crudely purified product may be a residue obtained by filtering a solid matter precipitated or sedimented from the reaction mixture.

As described above, the crudely purified product may be a liquid-form crude raw material at a room temperature (20°C) (such as an organic solvent solution such as the organic phase or the filtrate) containing the organic solvent and/or others, or may be a wet solid-form crude raw material at a room temperature (20°C) [such as a residue (a solid particle) separated by filtration of the precipitated solid matter].

A particularly preferred crudely purified product includes a dry solid (or a dry solid matter) of the crudely purified product, specifically a solid-form crudely purified product, containing 1,6-form, at a room temperature (20°C), for example, a dry or dried product obtained by removing the solvent from the above-mentioned liquid-form crude raw material (such as the organic solution such as the separated organic phase), and a dry or dried product obtained by drying or solidifying the wet solid at a room temperature (20°C).

The distillation condition of the crude raw material may use either a simple distillation (evaporation) or a continuous distillation, depending on the composition of the crude raw material (such as the species and contents of a low boiling point component and a high boiling point component), the form of the crude raw material (such as liquid or solid), and others. In the continuous distillation, a plate column (a tray column) or a packed column may be used, or a thin film distillation apparatus may be used. The number of theoretical plates of the distillation column may, for example, be about 0 to 100, preferably about 0 to 50, and further preferably about 1 to 10. In the distillation column, reflux may optionally be carried out, and the reflux ratio may, for example, be about 1 to 100, preferably about 5 to 50, and further preferably about 10 to 20. As described later, in a preferred embodiment, there may be used a distillation apparatus having a simple structure with about 0 to 5 distillation plates, particularly about 0 to 2 distillation plates, and especially a simple distillation apparatus having zero distillation plates.

The distillation (including the simple distillation) may be carried out, if necessary, in an air flow or under an atmosphere (or in a flow) of an inert gas (such as nitrogen gas and a rare or noble gas). In a preferred embodiment, the boiling point of 1,6-form is lower than those of the other positional isomers of naphthalenedithiol, while the boiling point of 1,6-form is still high. Thus, the crude raw material is distilled under a reduced pressure or under vacuum to obtain a distillate fraction containing 1,6-form. Specifically, in order to distill the crude raw material while preventing the production of and contamination with a polymeric 1,6-form, it is preferred to obtain a distillate fraction containing 1,6-form by distillation under a reduced pressure (or vacuum distillation). The pressure of the distillation under a reduced pressure (or the vacuum distillation) may be about 0.1 to 30 hPa, preferably about 1 to 25 hPa, further preferably about 5 to 20 hPa, and particularly about 10 to 20 hPa, depending on the distillation temperature (the temperature of the distillation apparatus).

In particular, for the distillation of the crude raw material, it is preferred to obtain the distillate fraction containing 1,6-form, under the above-mentioned pressure, at a temperature (or a column top temperature or a temperature of the distillate fraction) of about 140 to 212°C (such as about 145 to 210°C), preferably about 150 to 210°C, further preferably about 160 to 205°C, and particularly about 170 to 200°C (such as about 180 to 200°C). The temperature (or the column top temperature) of the distillate fraction containing 1,6-form may be about 140 to 200°C, preferably about 140 to 185°C, and further preferably about 140 to 170°C (such as about 140 to 160°C). In the distillation operation, the temperature (or the column top temperature) can be lowered as the pressure is decreased.

The distillation temperature (the internal temperature or column bottom temperature of the distillation apparatus) of the crude raw material is any temperature capable of obtaining (or withdrawing) the distillate fraction containing 1,6-form, and may be about 160 to 235°C, preferably about 170 to 230°C (such as about 180 to 225°C), further preferably about 190 to 225°C (such as about 195 to 225°C), and particularly about 200 to 220°C, depending on the pressure. The above-mentioned distillation temperature of the crude raw material may be about 160 to 220°C (such as about 160 to 205°C), preferably about 160 to 195°C (such as about 160 to 190°C), and further preferably about 160 to 180°C.

In a preferred process, the crude raw material may be distilled (distilled under a reduced pressure or under vacuum) at a distillation temperature (an internal temperature or column bottom temperature of the distillation apparatus) of 160°C to 235°C (such as 165 to 230°C) under a pressure of 0.1 to 30 hPa (such as 0.5 to 25 hPa) to obtain (or withdraw) a distillate fraction containing 1,6-form having a temperature of 140 to 212°C (such as 145 to 205°C), thus producing a 1,6-form product.

The distillation of the liquid crude raw material (such as the separated organic phase) or the wet solid crude raw material (such as the residue or cake obtained by filtering the precipitation product) allows a low boiling point component such as the organic solvent to be obtained from the column top of the distillation apparatus (a simple distillation apparatus or a continuous distillation apparatus) and allows a distillate fraction containing 1,6-form to be obtained from a middle position or a lower position (or a column bottom) of the distillation apparatus. The distillate fraction containing 1,6-form obtained from the middle position or the lower position of the distillation apparatus may further be distilled by a distillation apparatus (such as a simple distillation apparatus). Though such a process uses multiple distillation apparatuses, the distillations of the crudely purified product can significantly improve the purity of the 1,6-form product and prevent the 1,6-form product from the contamination with the colored component.

Thus, although the distillate fraction containing 1,6-form may be subjected to further purification, the present invention can achieve the production of a 1,6-form product having a high purity with little coloration by a single distillation operation (a distillation operation under a reduced pressure or vacuum), that is, a simple distillation under a reduced pressure or vacuum. Specifically, the continuous distillation of the dry solid of the crudely purified product by a simple distillation (a simple distillation apparatus) allows a distillate fraction containing 1,6-form to be obtained from an upper position (such as a column top) of the distillation apparatus (the evaporation apparatus) and allows a polymeric form such as a hardly volatile disulfide form to be removed from a lower position (or a column bottom) of the distillation apparatus (the evaporation apparatus). Moreover, in a batch operation, the polymeric form such as the disulfide form can remain in the lower position of the distillation apparatus. Thus, the process according to the present invention preferably contains at least a simple distillation operation, and such a simple operation as the simple distillation can effectively prevent the 1,6-form product from the contamination with the colored component and significantly improve the purity of the 1,6-form product. In other words, the 1,6-form product having a high purity with little coloration can be produced by simply withdrawing (or recovering) and cooling the above-mentioned distillate fraction.

To the simple distillation apparatus (the evaporation apparatus), the dry solid of the crudely purified product, which may optionally be molten, may be fed continuously for continuous distillation, or the dry solid of the crudely purified product may be fed batchwise for batch distillation. As the simple distillation apparatus (the evaporation apparatus), there may be used an apparatus equipped with an evaporator. Such an apparatus may comprise a heating (or heatable) evaporator, a feed line connected to the evaporator for feeding a dry solid of a crudely purified product in a molten state, a condenser connected to an upper position (or a head position) of the evaporator for withdrawing a distillate fraction containing 1,6-form, and a receiver for receiving a distillate withdrawn from the condenser. The simple distillation apparatus (the evaporation apparatus) may be a thin film distillation apparatus (evaporation apparatus) which distills a dry solid of a crudely purified product while forming a thin film (a thin film of a molten material) on an evaporation surface under a reduced pressure or under vacuum, or may be a falling thin-film evaporator, a centrifugal thin-film evaporator, and others. Such a distillation (or evaporation) can allow a smooth distillation (or evaporation) operation without maintaining the condenser or the receiver at a high temperature because of the low melting point of 1,6-form.

### [Naphthalene derivative]

The 1,6-form (or the 1,6-form product) according to the present invention has a high purity and a significantly reduced coloration which is due to a colored component, and further has a high refractive index and a high dissolubility in an organic solvent, and thus the 1,6-form (or the 1,6-form product) is useful as a reaction reagent, a polymer raw material, and others to prepare a naphthalene derivative (a derivative of 1,6-form). Such a naphthalene derivative also has or retains the characteristics of the 1,6-form (or the 1,6-form product), and has less coloration, a high refractive index, and a high dissolubility. For example, the 1,6-form (or the 1,6-form product) has an excellent dissolubility in an organic solvent compared with the other positional isomers (such as 1,5-, 2,6-, or 2,7-form), and can easily or efficiently be dissolved at a high concentration even under a room temperature (about 20°C) and can form a homogeneous (or uniform) solution. Thus, the applications of the 1,6-form (or the 1,6-form product) can be expanded significantly.

As examples of the organic solvent for the 1,6-form (or the 1,6-form product), there may be mentioned a hydrocarbon (such as an aromatic hydrocarbon such as toluene and xylene), a halogenated hydrocarbon (such as a chlorinated hydrocarbon such as methylene chloride and chloroform), an alcohol (such as isopropanol), an ether (such as tetrahydrofuran (THF) and 1,4-dioxane), a glycol ether (such as a cellosolve such as methyl cellosolve and ethyl cellosolve, and a carbitol such as methyl carbitol and ethyl carbitol), a glycol ether acetate (such as methyl cellosolve acetate, ethyl cellosolve acetate, methyl carbitol acetate, ethyl carbitol acetate, and propylene glycol monomethyl ether acetate (PGMEA)), a ketone (such as acetone and methyl ethyl ketone), an ester (such as an acetate ester such as ethyl acetate), a nitrile (such as acetonitrile), an amide (such as N,N-dimethylformamide (DMF) and N,N-dimethylacetamide (DMAc)), a urea (such as 1,3-dimethyl-2-imidazolidinone (DMI) and dimethylpropyleneurea (DMPU)), and a sulfoxide (such as dimethyl sulfoxide). Such an organic solvent may be used alone or as a mixed solvent in combination of two or more.

In addition, since 1,6-form and a derivative thereof have a high refractive index with the high dissolubility, use of 1,6-form and the derivative thereof as a polymer raw material can increase a content of the skeleton of 1,6-form in the resulting polymer to increase a refractive index of the polymer. Further, probably because 1,6-form has an asymmetric structure, a transparent polymer suitable as an optical material can be obtained. Thus, the 1,6-form (or the 1,6-form product) can also be used as various reaction reagents, polymer raw materials, and others, by using highly reactive mercapto groups. For example, 1,6-form can be used to prepare a polymer using a thiol-ene reaction. Further, the derivative of 1,6-form can also be used as a reaction reagent, a polymer raw material, and others by using a reactive group introduced to the derivative. In a preferred embodiment, 1,6-form and the derivative thereof can be used for an optical material.

Such a naphthalene derivative (the derivative of 1,6-form) can be represented by the following formula (2): 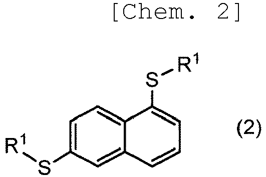
wherein R¹ represents any of groups represented by the following formulae (2a) to (2e): 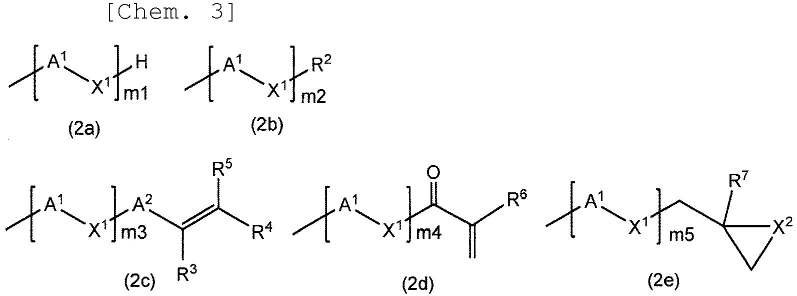
wherein A¹ represents an alkylene group, X¹ represents an oxygen atom or a sulfur atom, R² represents an alkyl group, A² represents a direct bond (a single bond) or an alkylene group, R³, R⁴, and R⁵ independently represent a hydrogen atom or an alkyl group, R⁶ and R⁷ each represent a hydrogen atom or a methyl group, X² represents an oxygen atom or a sulfur atom, m1 denotes an integer of not less than 1, and m2 to m5 each denote 0 or an integer of not less than 1.

In the formulae (2a) to (2e), A¹ may represent a C₁₋₆alkylene group (particularly a C₂₋₄alkylene group), and X¹ may represent an oxygen atom or a sulfur atom (particularly an oxygen atom).

As representative examples of the compound in which R¹ represents a group defined by the formula (2a), there may be mentioned a compound in which m1 in the formula (2a) denotes 1 to 4 (particularly 1 or 2), such as a 1,6-bis(hydroxyC₂₋₄alkylthio)naphthalene, a 1,6-bis(hydroxyC₂₋₄alkoxyC₂₋₄alkylthio)naphthalene, a 1,6-bis(mercaptoC₂₋₄alkylthio)naphthalene, and a 1,6-bis(mercaptoC₂₋₄alkylthioC₂₋₄alkylthio)naphthalene. A preferred compound may include a compound in which R¹ represents a hydroxyalkyl group, such as a 1,6-bis(hydroxyalkylthio)naphthalene [such as a 1,6-bis(hydroxyC₂₋₃alkylthio)naphthalene such as 1,6-bis(hydroxyethylthio)naphthalene].

Such a compound can be prepared by allowing 1,6-form to react with a reaction component selected from an alkylene oxide, an alkylene carbonate, a haloalkanol, an alkylene sulfide, an alkylene trithiocarbonate, and a haloalkanethiol in a solvent such as DMF and DMAc in the presence of a base such as an alkali metal hydroxide (such as sodium hydroxide). In order to prevent the production of a disulfide form as a by-product, the reaction may be carried out in the presence of a conventional reducing agent and/or polymerization inhibitor.

In the formula (2b), R² may represent a straight- or branched-chain C₁₋₄alkyl group. As representative examples of the compound in which R¹ represents a group defined by the formula (2b), there may be mentioned a compound in which m2 in the formula (2b) denotes 0 to 4 (particularly 0 to 2), such as a 1,6-bis(C₁₋₄alkylthio)naphthalene, a 1,6-bis(C₁₋₄alkoxyC₂₋₄alkylthio)naphthalene, and a 1,6-bis(C₁₋₄alkylthioC₂₋₄alkylthio)naphthalene. A preferred compound may include a compound in which R¹ represents an alkylthio group or an alkylthioalkyl group, such as a 1,6-bis(C₁₋₂alkylthio)naphthalene such as 1,6-bis(methylthio)naphthalene.

Such a compound can be prepared by allowing 1,6-form to react with an alkyl halide such as an alkyl iodide (such as methyl iodide and ethyl iodide) in a solvent such as methylene chloride, DMF, and DMAc in the presence of a base such as an alkali metal hydroxide (such as sodium hydroxide). In order to prevent the production of a disulfide form as a by-product, the reaction may be carried out in the presence of a conventional reducing agent and/or polymerization inhibitor.

As representative examples of the compound in which R¹ represents a group defined by the formula (2c), there may be mentioned a compound in which, in the formula (2c), m3 denotes 0 to 4 (particularly 0 to 2), and R³, R⁴, and R⁵ each represent a hydrogen atom or a methyl group, and preferably a compound in which the group defined by the formula (2c) represents a vinyl group or an allyl group. Such a compound may include a 1,6-bis(C₂₋₄alkenylthio)naphthalene, a 1,6-bis(C₂₋₃alkenyloxyC₂₋₃alkylthio)naphthalene, and a 1,6-bis(C₂₋₃alkenylthioC₂₋₃alkylthio)naphthalene, and examples of a preferred compound may include a 1,6-bis(C₂₋₃alkenylthio)naphthalene such as 1,6-bis(vinylthio)naphthalene, 1,6-bis(allylthio)naphthalene, and 1,6-bis(isopropenylthio)naphthalene.

These compounds can be prepared by allowing 1,6-form to react with an alkenyl halide such as an alkenyl iodide (such as a C₂₋₆alkenyl iodide such as allyl iodide and isopropenyl iodide) in a solvent such as methylene chloride, DMF, and DMAc in the presence of a base such as an alkali metal salt (such as sodium carbonate), and a polymerization inhibitor (such as a quinone such as methoquinone). The solvent may contain water.

As representative examples of the compound in which R¹ represents a group defined by the formula (2d), there may be mentioned a compound in which, in the formula (2d), m4 denotes 0 to 4 (particularly 0 to 2) and R⁶ represents a hydrogen atom or a methyl group, and preferably a compound in which the group defined by the formula (2d) represents a (meth)acryloyl group. Examples of such a compound may include a 1,6-bis[(meth)acryloyloxyC₂₋₃alkylthio]naphthalene such as 1,6-bis[(meth)acryloylthio]naphthalene and 1,6-bis[(meth)acryloyloxyethylthio]naphthalene; examples of a preferred compound may include 1,6-bis(methacryloylthio)naphthalene.

These compounds can be prepared by allowing 1,6-form to react with a (meth)acryloyl halide (preferably a methacryloyl halide) such as (meth)acryloyl chloride and (meth)acryloyl bromide in a solvent such as methylene chloride, DMF and DMAc in the presence of a polymerization inhibitor (such as a quinone such as methoquinone).

As representative examples of the compound in which R¹ represents a group defined by the formula (2e), there may be mentioned a compound in which, in the formula (2e), m4 denotes 0 to 4 (particularly 0 to 2), R⁷ represents a hydrogen atom or a methyl group (particularly a hydrogen atom), and X² represents an oxygen atom or a sulfur atom (particularly an oxygen atom), and preferably a compound in which the group defined by the formula (2d) represents a glycidyl group or a glycidyloxyC₂₋₃alkyl group. Examples of such a compound may include a 1,6-bis(glycidyloxyC₂₋₃alkylthio)naphthalene such as 1,6-bis(glycidylthio)naphthalene and 1,6-bis(glycidyloxyethylthio)naphthalene. The compound having the group represented by the formula (2e) may be a monomer or may contain a polymeric form such as from a dimer to a decamer.

Such a compound can be prepared by allowing 1,6-form to react with an epihalohydrin such as epichlorohydrin, and/or an epithiohalohydrin such as epithiochlorohydrin in the presence of a phase transfer catalyst such as benzyltributylammonium chloride.

A preferred derivative of 1,6-form may be a derivative in which R¹ in the formula (2) is at least one member selected from a hydroxyalkyl group, an alkyl group, an alkenyl group (such as vinyl group and allyl group), a (meth)acryloyl group, a (meth)acryloyloxyalkyl group, a glycidyl group, and a glycidyloxyalkyl group. Among these derivatives of 1,6-form, a derivative having a reactive group such as a hydroxyalkyl group can be used as a monomer for a polymer, and a derivative having a polymerizable or curable group such as (meth)acryloyl group and glycidyl group is useful for forming a curable resin or a curable composition containing the curable resin.

### EXAMPLES

The following examples are intended to describe this invention in further detail and should by no means be interpreted as defining the scope of the invention. The evaluation methods were shown below.

### [¹H-NMR]

The ¹H-NMR was measured using "JNM-ECZ400 (400 MHz)" manufactured by JEOL Ltd. and "JNM-ECA600 (600 MHz)" manufactured by JEOL Ltd., and using chloroform-d (CDCl₃) as a deuterated solvent and tetramethylsilane (TMS) as a standard substance.

### [HPLC purity, disulfide form content (dimeric form content)]

The area normalization (or area percentage) by HPLC measured under the following conditions was determined as a HPLC purity and a disulfide form (dimeric form) content. The disulfide form (dimeric form) content less than a detection limit was expressed as "N.D.".
HPLC Apparatus: "LC-20A" manufactured by SHIMADZU CORPORATION
Column: YMC-Triart C18 (5 um, 4.6 mmϕ x 150 mm)
Mobile phase: 5% phosphoric acid aqueous solution/acetonitrile (acetonitrile from 65% to 90%)
Flow rate: 1.0 ml/min, column temperature: 40°C, detection wavelength: UV 210 nm

### [Melting point]

The melting point was measured using a melting point measuring apparatus ("535" manufactured by BUCHI) in accordance with Japanese Industrial Standards (JIS) K 4101 (1993) [5.1 Method according to Visual Observation].

### [Gardner color scale]

A sample in a molten state maintained at 50°C was visually compared with Gardner color scale standard solutions, and the corresponding color scale of the sample was determined.

### [Refractive index]

The refractive index of each sample was measured using an abbe refractometer ("NAR-1T" manufactured by ATAGO CO., LTD.) at a temperature of 25°C and a wavelength of 589 nm. Depending on the species of each sample, the refractive index was measured as follows.

### [Dissolubility]

In each of predetermined solvents described in the Table below, a sample was mixed at a temperature of 25°C, and the dissolubility of the sample was evaluated. In more detail, the sample was added to a predetermined amount of a solvent until the sample could not be dissolved, and the sample content of the liquid phase (the solvent phase) of the resulting mixture was analyzed using an HPLC ("SPD-20A" manufactured by SHIMADZU CORPORATION), and thus the concentration (the maximum dissolved concentration) of the sample in the liquid phase (the solvent phase) was determined as a solubility.

### [Example 1]

### [Synthesis of 1,6-naphthalenedithiol (1,6-form)]

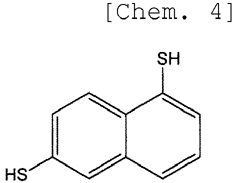

In a 1000 mL flask, 100 g (300.9 mmol) of disodium 1,6-naphthalenedisulfonate, 451 g of toluene, 8.8 g (0.4 molar ratio) of N,N-dimethylformamide, and 89.6 g (2.5 molar ratio) of thionyl chloride were charged under a nitrogen atmosphere, and the mixture was heated to 100°C and subjected to a reaction at a temperature of 97 to 102°C for 8 hours. After the completion of the reaction, the reaction mixture was cooled to 30°C, and 451 g of water was added to the cooled mixture at 20 to 30°C, and the resulting mixture was stirred for 10 minutes or more. The resulting lower aqueous phase was removed by liquid-liquid separation (or liquid-liquid phase separation). To the obtained organic phase was added 451 g of water in the same manner as described above, the resulting mixture was stirred for 10 minutes or more and was subjected to liquid-liquid separation to obtain, as an organic phase, 622.5 g of a toluene solution containing 1,6-naphthalenedisulfonyl chloride (16NDSC) (purity by HPLC: 98%, theoretical yield: 97 mol%).

In a 5000 mL flask, 1800 g of toluene and 1439.8 g (48.8 molar ratio) of 36% by mass hydrochloric acid were charged under a nitrogen atmosphere, and the mixture was heated to 60°C. After the heating, 622.5 g (291.0 mmol) of the above-mentioned 16NDSC toluene solution and 258.8 g (13.6 molar ratio) of zinc powder were dividedly added to the heated mixture at a temperature of 60 to 70°C over 3 hours and 30 minutes, and the reaction was carried out at the above-mentioned temperature for one hour. After the completion of the reaction, the reaction mixture was cooled to 30°C and was allowed to stand at 0 to 30°C. Then, the resulting lower aqueous phase was removed by liquid-liquid separation. Thereafter, the obtained organic phase was subjected to filtration, and the residue was washed with 33 g of toluene to obtain a filtrate containing the washed toluene solution obtained by washing. The obtained filtrate was subjected to desolvation and drying at not higher than 45°C under a reduced pressure to obtain 50.5 g of a solid matter containing 1,6-naphthalenedithiol (1,6-form) (HPLC: purity 98.3%, disulfide form 1.4%, Gardner color scale: 5, theoretical yield: 90 mol%).

### [Distillation operation]

The resulting solid matter (148 g) containing 1,6-naphthalenedithiol (1,6-form) was charged to a distillation apparatus (a simple distillation apparatus) and was distilled at an internal temperature of 162 to 167°C and a pressure of 0.6 hPa to obtain 144 g of a colorless distillate fraction from a column top with a column top temperature (a top temperature) of 141 to 146°C (yield: 97 mol%, HPLC: purity 100.0%, disulfide form "N.D.", Gardner color scale: less than 1).

Melting point: 35 to 36°C
Refractive index: 1.722
¹H-NMR (CDCl₃): δ (ppm) 3.57 (s, 1H), 3.61 (s, 1H), 7.31 (dd, J 8.4 7.4, 1H), 7.40 (dd, J 9.0 1.8, 1H), 7.48 (d, J 7.2, 1H), 7.55 (d, J 8.4, 1H), 7.72 (d, J 1.8, 1H), 8.03 (d, J 9.0, 1H)

The following Table shows the evaluation results of the 1,6-form product obtained by the distillation operation of Example 1.

### [Table 1]

**Table 1**

| | Item | HPLC area fraction % | | Gardner color scale |
|---|---|---|---|---|
| | | Purity | Disulfide form content | |
| 1,6-Form product | Before distillation | 98.3 | 1.4 | 5 |
| | After distillation | 100.0 | N.D. | less than 1 |

### Examples 2 to 6

In each Example, the distillation was carried out in the same manner as the distillation operation of Example 1 except that the distillation was carried out under the following conditions, thus obtaining a 1,6-naphthalenedithiol product. The resulting 1,6-naphthalenedithiol products each were as follows: HPLC: purity 100.0%, disulfide form "N.D.", Gardner color scale: less than 1.
Example 2: an internal temperature of 167°C, a pressure of 1 hPa, and a column top temperature (a top temperature) of 146°C
Example 3: an internal temperature of 200°C, a pressure of 10 hPa, and a column top temperature (a top temperature) of 181°C
Example 4: an internal temperature of 208°C, a pressure of 15 hPa, and a column top temperature (a top temperature) of 187°C
Example 5: an internal temperature of 218°C, a pressure of 20 hPa, and a column top temperature (a top temperature) of 194°C
Example 6: an internal temperature of 234°C, a pressure of 30 hPa, and a column top temperature (a top temperature) of 211°C

### [Comparative Example 1] Crystallization of 1,6-form from methanol

In a 1 L flask, 25.0 g of a crudely purified product of 1,6-naphthalenedithiol (HPLC: purity 98.9%, disulfide form 1.1%, Gardner color scale: 5) and 250.0 g of methanol were charged, and the mixture was stirred at 25°C to dissolve the crudely purified product in methanol. The resulting solution was cooled to not higher than 5°C, 44.1 g of water was dropwise added thereto to precipitate a crystal, and the resulting precipitate was collected by filtration to obtain 18.1 g of a yellow solid matter (yield: 71 mol%, HPLC: purity 99.3%, disulfide form 0.7%, Gardner color scale: 5).

The crude raw material of 1,6-naphthalenedithiol was obtained by repeating the same reaction as the above-mentioned Example 1. In this example, since crystallization from methanol did not improve the degree of coloration, the purification operation was stopped after one crystallization operation.

### [Comparative Example 2] Crystallization of 1,6-form from heptane

In a 1 L flask, 20.0 g of a crudely purified product of 1,6-naphthalenedithiol (HPLC: purity 98.9%, disulfide form 1.1%, Gardner color scale: 5) and 229.5 g of heptane were charged, and the mixture was heated to 98°C under stirring. After the stirring, the mixture was slowly cooled to not higher than 5°C to precipitate a crystal, and the resulting precipitate was collected by filtration to obtain 7.9 g of a yellow solid matter (yield: 40 mol%, HPLC: purity 98.9%, disulfide form 1.1%, Gardner color scale: 4).

The resulting yellow solid matter (7.9 g) and heptane (90.9 g) were charged, and the mixture was heated to 98°C under stirring. After the stirring, the mixture was slowly cooled to 5°C to precipitate a crystal, and the resulting precipitate was collected by filtration to obtain 5.9 g of a yellow solid matter (yield: 30 mol%, HPLC: purity 98.9%, disulfide form 1.1%, Gardner color scale: 4).

As described above, although the crystallization operation from heptane was carried out twice, a 1,6-form product having a high purity with less or no coloration was not obtained. In particular, there was no change in Gardner color scale even after the crystallization twice.

### [Comparative Example 3] Purification of 1,6-form by silica gel chromatography

A crudely purified product of 1,6-naphthalenedithiol (20.0 g) (HPLC: purity 98.9%, disulfide form 1.1%, Gardner color scale: 5) was separated and purified by silica gel column chromatography (ethyl acetate/heptane (volume ratio) = 1/32) to obtain 14.2 g of a light-yellow solid matter containing 1,6-naphthalenedithiol (theoretical yield: 72 mol%, HPLC: purity 99.6%, disulfide form: 0.3%, Gardner color scale: 3).

The resulting solid matter was separated and purified again by silica gel column chromatography (ethyl acetate/heptane (volume ratio) = 1/32) to obtain 10.0 g of a light-yellow solid matter containing 1,6-naphthalenedithiol (theoretical yield: 50 mol%, HPLC: purity 99.5%, disulfide form 0.3%, Gardner color scale: 3).

As described above, even though the purification operation was carried out twice by chromatography, it was difficult to increase the purity and reduce the disulfide form content, and also to remove a colored component.

### [Comparative Example 4] Adsorption treatment

In a 1 L flask, 25 g of a crudely purified product of 1,6-naphthalenedithiol (HPLC purity: 100.0%, disulfide form "N.D.", Gardner color scale: 5) and 250 g (10 times as much as the crudely purified product of 1,6-naphthalenedithiol) of methanol were charged under a nitrogen atmosphere, and the mixture was heated to 30°C. The mixture was stirred for 10 minutes or more, and complete dissolution was confirmed. To the resulting solution was added 2.5 g (10% by mass relative to the crudely purified product of 1,6-naphthalenedithiol) of an activated carbon ("SHIRASAGI A" manufactured by Osaka Gas Chemicals Co., Ltd.), and the mixture was stirred for 30 minutes and was then subjected to filtration. The resulting residue was washed with methanol to obtain 280.2 g of a 1,6-naphthalenedithiol solution A (HPLC purity: 99.0%, disulfide form: 1.0%), from which a colored component could not be removed. To the resulting 1,6-naphthalenedithiol solution A was added 2.5 g (10% by mass relative to the crudely purified product of 1,6-naphthalenedithiol) of an acid clay ("MIZUKALIFE F-1G" manufactured by Mizusawa Industrial Chemicals, Ltd.), and the mixture was stirred for 30 minutes or more. Thereafter, the mixture was subjected to filtration, and the resulting residue was washed with methanol to obtain 282.2 g of a 1,6-naphthalenedithiol solution B (HPLC purity: 98.5%, disulfide form: 1.5%), from which a colored component could not be removed. To the resulting solution B was added again 2.5 g (10% by mass relative to the crudely purified product of 1,6-naphthalenedithiol) of the above-mentioned acid clay. The mixture was stirred for 30 minutes or more and was then subjected to filtration, and the resulting residue was washed with methanol to obtain 282.2 g of a 1,6-naphthalenedithiol solution C (HPLC purity: 98.4%, disulfide form: 1.6%), from which a colored component could not be removed.

As described above, even though the decoloration with the activated carbon and the acid clay was carried out, it was difficult to increase the purity and reduce the disulfide form content, and also to remove a colored component.

### [Comparative Example 5] Synthesis of 1,5-form

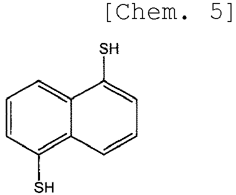

In a 5 L flask, 100 g (300.9 mmol) of disodium 1,5-naphthalenedisulfonate, 2003 g of toluene, 8.8 g (0.4 molar ratio) of N,N-dimethylformamide, and 85.9 g (2.4 molar ratio) of thionyl chloride were charged under a nitrogen atmosphere, and the mixture was heated to 100°C. After the heating, the temperature fluctuated from 97 to 102°C, and the reaction was carried out for 5 hours while maintaining the temperature in a thermostatic bath. After the completion of the reaction, the reaction mixture was cooled to 30°C, and 451 g of water was added thereto at 20 to 30°C, and the resulting mixture was stirred for 10 minutes or more. The resulting lower aqueous phase was removed by liquid-liquid separation. In the same manner, 150 g of water was added to the obtained organic phase. The resulting mixture was stirred for 10 minutes or more, and an organic phase was obtained by liquid-liquid separation. The organic phase was subjected to filtration, and the residue was washed with 30 g of toluene to obtain 2141.6 g of a toluene solution containing 1,5-naphthalenedisulfonyl chloride (15NDSC) (HPLC: purity 96%, theoretical yield: 96 mol%).

In a 10 L flask, 580 g of toluene and 1422.1 g (48.4 molar ratio) of 36% by mass hydrochloric acid were charged under a nitrogen atmosphere, and the mixture was heated to 60°C. After the heating, 2141.6 g (289.8 mmol) of the above-mentioned 15NDSC toluene solution and 257.7g (13.6 molar ratio) of zinc powder were dividedly added to the heated mixture over one hour and 30 minutes. The temperature during the addition was 60 to 70°C, and the reaction was carried out for one hour while maintaining the temperature in a thermostatic bath. After the completion of the reaction, the reaction mixture was cooled to 30°C and was allowed to stand at 0 to 30°C, and the resulting lower aqueous phase was removed by liquid-liquid separation. Thereafter, the obtained organic phase was subjected to filtration, and the residue was washed with 188 g of toluene. The resulting filtrate containing the washed toluene solution obtained by washing was subjected to desolvation and drying at not higher than 40°C under a reduced pressure to obtain 58.5 g of a solid matter containing 1,5-naphthalenedithiol (15-form) (HPLC: purity 99.4%, disulfide form "N.D.", theoretical yield: 99 mol%).

### [Distillation operation]

In a distillation apparatus (a simple distillation apparatus), 10 g of the solid matter containing 1,5-naphthalenedithiol (15-form) was charged and was distilled to obtain a distillate fraction having a boiling point 150°C/0.6 hPa. However, the distillate fraction was crystallized inside a condenser (melting point of 1,5-form: 120°C) and clogged an outflow line. Thus, the distillation operation was discontinued.

The characteristics of the resulting 1,5-form product were as follows. The 1,5-form product had a high melting point and was difficult to melt, and thus the Gardner color scale could not be measured. The 1,5-form product was yellow in color.

Melting point 120 to 121°C
Refractive index 1.711
¹H-NMR (CDCl₃): δ (ppm) 3.62 (s, 2H), 7.40 (dd, J 8.4 7.2, 2H), 7.59 (d, J 7.2, 2H), 8.04 (d, J 8.4, 2H)

### [Comparative Example 6] Synthesis of 2,6-form

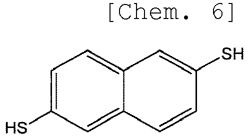

In a 1 L flask, 100 g (300.9 mmol) of disodium 2,6-naphthalenedisulfonate, 451 g of toluene, 8.8 g (0.4 molar ratio) of N,N-dimethylformamide, and 100.3 g (2.8 molar ratio) of thionyl chloride were charged under a nitrogen atmosphere, and the mixture was heated to 100°C. After the heating, the temperature fluctuated from 97 to 102°C, and the reaction was carried out for 8 hours while maintaining the temperature in a thermostatic bath. After the completion of the reaction, the reaction mixture was cooled to 30°C, and 226 g of water was added thereto at 20 to 30°C, and the resulting mixture was stirred for 2 hours or more and was then subjected to filtration. The resulting wet crystal was washed with 60 g of toluene and was dried to obtain 92.6 g of 2,6-naphthalenedisulfonyl chloride (26NDSC) as a solid matter (HPLC: purity 98%, theoretical yield: 93 mol%) .

In a 10 L flask, 4478 g of toluene and 249.0 g (13.6 molar ratio) of zinc powder were charged under a nitrogen atmosphere, and the mixture was heated to 60°C. After the heating, 92.6 g (279.9 mmol) of 26NDSC and 692.4 g (24.4 molar ratio) of 36% by mass hydrochloric acid were dividedly added to the heated mixture over one hour. The temperature during the addition was 60 to 70°C, and the reaction was carried out for 2 hours while maintaining the temperature in a thermostatic bath. After the completion of the reaction, the reaction mixture was subjected to filtration, and the resulting residue was washed with 55 g of toluene that was pre-heated to 60°C. The resulting filtrate containing the washed toluene solution obtained by washing was subjected to liquid-liquid separation at 55 to 60°C, then removing a lower aqueous phase. Thereafter, 221 g of water and 21.0 g (0.74 molar ratio) of 36% by mass hydrochloric acid were added to the obtained organic phase, and the mixture was heated to 50°C. After the heating, the temperature fluctuated from 50 to 60°C, and the mixture was stirred for 30 minutes while maintained at the above-mentioned temperature in a thermostatic bath. Then the resulting lower aqueous phase was removed by liquid-liquid separation. The obtained organic phase was subjected to desolvation to 398 g at not higher than 60°C under a reduced pressure, and the desolvated mixture was cooled to 5°C. After the cooling, the temperature fluctuated from 0 to 5°C, and the desolvated mixture was stirred for one hour while maintained at the above-mentioned temperature in a thermostatic bath, and then was subjected to filtration. The resulting wet crystal was washed with 55 g of water and was dried to obtain 40.2 g of a solid matter containing 2,6-naphthalenedithiol (26-form) (HPLC: purity 100%, disulfide form "N.D.", theoretical yield: 75 mol%).

### [Distillation operation]

In a distillation apparatus (a simple distillation apparatus), 24 g of the solid matter containing 2,6-naphthalenedithiol (2,6-form) was charged and was distilled, and sublimation started at 145°C/0.7 hPa (melting point of 2,6-form: 196 to 197°C) and caused an outflow line to be clogged. Thus, the distillation operation was discontinued.

The characteristics of the resulting 2,6-form product were as follows. The 2,6-form product had a high melting point and was difficult to melt, and thus the Gardner color scale of the 2,6-form product could not be measured. The 2,6-form product was yellow in color.

Melting point 196 to 197°C
Refractive index 1.718
¹H-NMR (CDCl₃): δ (ppm) 3.58 (s, 2H), 7.32 (dd, J 8.0 1.6, 2H), 7.57 (d, J 8.0, 2H), 7.68 (d, J 1.6, 2H)

### [Comparative Example 7] Synthesis of 2,7-form

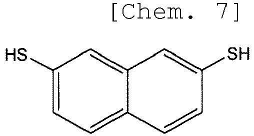

In a 1 L flask, 100 g (300.9 mmol) of disodium 2,7-naphthalenedisulfonate, 632 g of toluene, 8.8 g (0.4 molar ratio) of N,N-dimethylformamide, and 89.5 g (2.5 molar ratio) of thionyl chloride were charged under a nitrogen atmosphere, and the mixture was heated to 100°C. After the heating, the temperature fluctuated from 97 to 102°C, and the reaction was carried out for 3 hours while maintaining the temperature in a thermostatic bath. After the completion of the reaction, the reaction mixture was cooled to 30°C, and 451 g of water was added thereto at 20 to 30°C, and the resulting mixture was stirred for 10 minutes or more. The resulting lower aqueous phase was removed by liquid-liquid separation. In the same manner, 451 g of water was added to the obtained organic phase, and the resulting mixture was stirred for 10 minutes or more and was subjected to liquid-liquid separation to obtain, as an organic phase, 738.6 g of a toluene solution containing 2,7-naphthalenedisulfonyl chloride (27NDSC) (HPLC: purity 99%, theoretical yield: 99 mol%).

In a 10 L flask, 6507 g of toluene and 1264.3 g (41.9 molar ratio) of 36% by mass hydrochloric acid were charged under a nitrogen atmosphere, and the mixture was heated to 60°C. After the heating, 738.6 g (297.6 mmol) of the 27NDSC toluene solution and 264.7 g (13.6 molar ratio) of zinc powder were dividedly added to the heated mixture over 3 hours and 30 minutes. The temperature during the addition was 60 to 70°C, and the reaction was carried out for one hour while maintaining the temperature in a thermostatic bath. After the completion of the reaction, the reaction mixture was cooled to 30°C and was allowed to stand at 0 to 30°C, and the resulting lower aqueous phase was removed by liquid-liquid separation. Thereafter, the obtained organic phase was subjected to filtration, and the residue was washed with 75 g of toluene. The resulting filtrate containing the washed toluene solution obtained by washing was subjected to desolvation to 203 g at not higher than 55°C under a reduced pressure, and the desolvated mixture was subjected to filtration. The resulting wet crystal was washed with 59 g of toluene and was dried to obtain 43.1 g of a solid matter containing 2,7-naphthalenedithiol (27-form) (HPLC: purity 99.5%, disulfide form "N.D.", theoretical yield: 75 mol%).

### [Distillation operation]

In a distillation apparatus, 20 g of the solid matter containing 2,7-naphthalenedithiol (2,7-form) was charged and was distilled, and sublimation started at 145°C/0.7 hPa (melting point of 2,7-form: 186 to 188°C) and caused an outflow line to be clogged. Thus, the distillation operation was discontinued.

The characteristics of the resulting 2,7-form product were as follows. The 2,7-form product had a high melting point and was difficult to melt, and thus the Gardner color scale of the 2,7-form product could not be measured. The 2,7-form product was yellow in color.

Melting point 186 to 188°C
Refractive index 1.721
¹H-NMR (CDCl₃): δ (ppm) 3.60 (s, 2H), 7.27 (dd, J 8.4 1.2, 2H), 7.58 (d, J 1.2, 2H), 7.64 (d, J 8.4, 2H)

The following Table shows the evaluation results of the naphthalenedithiol products obtained in Example 1 and Comparative Examples 6 to 8. In the Table, "THE" means tetrahydrofuran, "DMF" means N,N-dimethylformamide, and "PGMEA" means propylene glycol monomethyl ether acetate (the same applies hereinafter). As described above, the Gardner color scale of the 1,5-form product, the 2,6-form product, and the 2,7-form product could not be measured.

### [Table 2]

**Table 2**

| | | Refractive index | Dissolubility [% bv mass] | | | | | |
|---|---|---|---|---|---|---|---|---|
| | | | Methanol | Toluene | THF | DMF | Ethyl acetate | PGMEA |
| Example 1 | 1,6-Form product | 1.722 | 19.3 | Freely dissolved (>90) | | | | 85.3 |
| Comparative Example 5 | 1,5-Form product | 1.711 | 1.08 | 7.26 | 29.0 | 34.4 | 7.05 | 7.44 |
| Comparative Example 6 | 2,6-Form product | 1.718 | 0.11 | 0.64 | 4.03 | 3.54 | 0.65 | 0.73 |
| Comparative Example 7 | 2,7-Form product | 1.721 | 0.16 | 0.76 | 5.24 | 7.75 | 0.90 | 1.05 |

As apparent from Table 2, among Comparative Examples, Comparative Example 6 (the 1,5-form product), which had the lowest refractive index, had the highest dissolubility in all solvents. On the other hand, Example 1 (the 1,6-form product) had both high refractive index and high dissolubility compared with Comparative Example 6 (the 1,5-form product), in particular, had a significantly high dissolubility. Specifically, Example 1 (the 1,6-form product) was dissolvable even at a concentration of not less than 90% by mass in each one of toluene, THE, DMF, and ethyl acetate, and even at a concentration of not less than 85% by mass in PGMEA.

### INDUSTRIAL APPLICABILITY

The 1,6-naphthalenedithiol (or the 1,6-naphthalenedithiol product) according to the present invention has a high purity and a significantly reduced coloration. In addition, the 1,6-naphthalenedithiol (or the 1,6-naphthalenedithiol product) has both high dissolubility and high refractive index. Thus, the 1,6-naphthalenedithiol (or the 1,6-naphthalenedithiol product) according to the present invention can be used for various purposes including a reagent, a raw material or reaction intermediate for a functional compound, an optical material [such as a display material such as an anti-reflective film (such as an index matching film), and an additive (or a resin additive) such as a refractive index increasing agent, a fluorescence quencher, a crosslinking agent, or a crosslinking coagent], an electric and electronic material (such as a resist material such as a resist under-layer, and an organic semiconductor material), and a coating material or a coat material (such as a coating composition for forming a hardcoat layer and/or others). The 1,6-naphthalenedithiol (or the 1,6-naphthalenedithiol product) according to the present invention can also be used as, for example, a resin raw material or a monomer component (such as a monomer component having a high refractive index). As examples of the resin which can be prepared from the above-mentioned resin raw material or monomer component, there may be mentioned a thermosetting or photo-curable resin such as a (meth)acrylic resin, an epoxy-series resin, and a diallyl-series resin; and a thermoplastic resin such as a polyester-series resin (such as a polyester resin, a polyester carbonate resin, a polycarbonate resin, and a polythioester resin), a polyether-series resin, a polysulfide-series resin (such as a polyaddition product by thiol-ene reaction), and a polyurethane-series resin (such as a polyurethane resin and a polythiourethane resin).

## Claims

1. A 1,6-naphthalenedithiol product having a purity of not less than 99.5% and a polymeric 1,6-naphthalenedithiol content of not more than 0.2%, each determined by high performance liquid chromatography using area normalization, and having a Gardner color scale in a molten state of not more than 1.

2. The 1,6-naphthalenedithiol product according to claim 1, wherein the polymeric 1,6-naphthalenedithiol is a disulfide form as a dimeric 1,6-naphthalenedithiol.

3. A process for producing a 1,6-naphthalenedithiol product from a crude raw material containing 1,6-naphthalenedithiol and at least a polymeric 1,6-naphthalenedithiol, wherein the process comprises distilling the crude raw material.

4. The process according to claim 3, wherein the crude raw material is a crudely purified product of 1,6-naphthalenedithiol from a reaction mixture which is obtained in synthesis of 1,6-naphthalenedithiol.

5. The process according to claim 3 or 4, wherein the crude raw material is a dry solid of a crudely purified product of 1,6-naphthalenedithiol from a reaction mixture which is obtained in synthesis of 1,6-naphthalenedithiol.

6. The process according to any one of claims 3 to 5, which comprises subjecting the crude raw material to a distillation under a reduced pressure or a vacuum distillation to obtain a distillate fraction containing 1,6-naphthalenedithiol.

7. The process according to any one of claims 3 to 6, which comprises distilling the crude raw material at a pressure of 0.1 to 30 hPa to obtain a distillate fraction having a temperature of 140 to 212°C.

8. The process according to any one of claims 3 to 7, which comprises subjecting the dry solid of the crudely purified product recited in claim 4 as the crude raw material to a simple distillation to obtain a distillate fraction containing 1,6-naphthalenedithiol from an upper position of a distillation apparatus.
